(19) Europäisches Patentamt
European Patent Office
Office européen des brevets

(11) EP 3 761 004 A1

# (12) EUROPEAN PATENT APPLICATION
published in accordance with Art. 153(4) EPC

(43) Date of publication:
06.01.2021 Bulletin 2021/01

(21) Application number: 19760174.3

(22) Date of filing: 25.01.2019

(51) Int Cl.:
G01N 15/14 (2006.01)      G01N 33/543 (2006.01)

(86) International application number:
PCT/JP2019/002505

(87) International publication number:
WO 2019/167499 (06.09.2019 Gazette 2019/36)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.02.2018 JP 2018033515

(71) Applicant: SYSMEX CORPORATION
Kobe-shi
Hyogo 651-0073 (JP)

(72) Inventor: TABATA Seiichiro
Kobe-shi, Hyogo 651-0073 (JP)

(74) Representative: Hoffmann Eitle
Patent- und Rechtsanwälte PartmbB
Arabellastraße 30
81925 München (DE)

## (54) PARTICLE MEASURING DEVICE AND PARTICLE MEASURING METHOD

(57) Provided are a particle measuring device and a particle measuring method that can omit the work for position adjustment of optical fibers and maintain a state where light appropriately enters the optical fibers. A particle measuring device (10) includes: a flow cell (20) in which a sample (11) including a particle flows; an irradiator (30) configured to irradiate an irradiation light to the sample (11) flowing in the flow cell (20); a condenser lens (42) to condense a light generated from the particle included in the sample (11) which is irradiated by the irradiation light; a light transmitter (50) which is formed by a plurality of optical fibers (51) being bundled, and which the light having passed through the condenser lens (42) enters; and a light detector (61) configured to receive the light transmitted by the light transmitter (50) and output a detection signal.

FIG.1

EMBODIMENT 1

**Description**

TECHNICAL FIELD

The present invention relates to a particle measuring device and a particle measuring method for measuring particles.

BACKGROUND ART

A flow cytometer that applies laser light to a particle, and guides light generated from the particle to a detector through an optical fiber is known. For example, PATENT LITERATURE 1 describes multi-color flow cytometry that analyzes a plurality of fluorescence signals obtained from a plurality of types of target substances in a biological sample.

Specifically, as shown in FIG. 14, PATENT LITERATURE 1 describes an optical unit that includes: laser light sources 601, 602, 603, 604 that emit laser lights having different wavelengths from one another; a flow cell 610 to which the laser lights are applied; a condenser lens 620 that condenses fluorescences generated by application of the laser lights; detectors 631, 632, 633, 634; optical fibers 641 642, 643, 644 that respectively guide fluorescences condensed by the condenser lens 620 to the detectors 631, 632, 633, 634; and a holder 650 that supports the optical fibers 641, 642, 643, 644. In addition, PATENT LITERATURE 1 indicates that: fluorescence generated by application of laser light of one type is guided by one optical fiber to a detector; and when fluorescence is deviated from a light-entry-side end surface of the optical fiber, alignment of the optical fiber is performed by moving the holder.

CITATION LIST

[PATENT LITERATURE]

[PTL 1] US Patent No. 6683314

SUMMARY OF THE INVENTION

PROBLEMS TO BE SOLVED BY THE INVENTION

However, the diameter of one optical fiber is as small as 9 $\mu$m to 60 $\mu$m, in general. Thus, in the case of the optical unit as described above, even when the optical fiber is slightly displaced, light does not appropriately enter the optical fiber. Accordingly, the accuracy of a measurement result is reduced. In the aforementioned multi-color flow cytometry, in order to simultaneously and accurately detect a plurality of fluorescences from a large number of target substances, it is necessary to perform position adjustment of the optical fibers every time a measurement is performed. However, if position adjustment is required for each measurement, work burden for the maintenance on a user is increased, and in addition, the user is required to have proficiency for performing the maintenance.

SOLUTION TO THE PROBLEMS

A first aspect of the present invention relates to a particle measuring device. A particle measuring device (10) according to the present aspect includes: a flow cell (20) in which a sample (11) including a particle flows; an irradiator (30) configured to irradiate an irradiation light to the sample (11) flowing in the flow cell (20); a condenser lens (42) to condense a light generated from the particle included in the sample (11) which is irradiated by the irradiation light; a light transmitter (50, 210, 220, 230) which is formed by a plurality of optical fibers (51, 211, 221, 231) being bundled, and which the light having passed through the condenser lens (42) enters; and a light detector (61, 300, 400, 500) configured to receive the light transmitted by the light transmitter (50, 210, 220, 230) and output a detection signal.

According to the particle measuring device of the present aspect, the light condensed by the condenser lens is guided to the light transmitter formed by a plurality of optical fibers being bundled. In this case, the diameter of the light-entry-side end surface of the light transmitter can be easily set to be greater than the diameter of the luminous flux condensed by the condenser lens. Accordingly, the light condensed by the condenser lens can easily enter the light-entry-side end surface of the light transmitter. Thus, even when a slight displacement of the optical fibers has occurred, the light generated from the particle can be inhibited from being deviated from the light transmitter. Thus, the work of the user for position adjustment of the optical fibers can be omitted, and a state where the light appropriately enters the optical fibers can be maintained.

In the particle measuring device (10) according to the present aspect, the condenser lens (42) may condense the light generated from the particle, within a light-entry-side end surface (50a, 210a, 220a, 230a) of the light transmitter (50, 210, 220, 230). With this configuration, even when displacement of the optical fibers has occurred, the state in which the light appropriately enters the optical fibers can be maintained.

In the particle measuring device (10) according to the present aspect, the condenser lens (42) may condense the light generated from the particle to a light-entry-side end surface (50a, 210a, 220a, 230a) of the light transmitter (50, 210, 220, 230), such that the light has an area having a diameter greater than that of a light entry end of each of the optical fibers (51, 211, 221, 231) and extending across a plurality of light entry ends of a plurality of the optical fibers (51, 211, 221, 231).

With this configuration, when compared with a case where light condensed by the condenser lens is condensed to the light-entry-side end surface of the light transmitter in a state where the light is narrowed to have a small area, the amount of light guided to the optical fibers in the light transmitter can be maintained at a constant level. That is, the light in a state of being narrowed to have a small area could enter only the cladding portion of an optical fiber or a gap between a plurality of bundled optical fibers. In this case, the amount of light guided to the light detector in the subsequent stage by the light transmitter is significantly reduced. However, when the light condensed by the condenser lens enters the light-entry-side end surface of the light transmitter such that the light has an area having a diameter greater than that of the light entry end of each optical fiber and extending across light entry ends of a plurality of optical fibers, a part of the light enters the cladding portion or the gap, but the other part of the light enters the core portions of the optical fibers. Accordingly, variation in the amount of light guided to the light detector in the subsequent stage by the light transmitter can be suppressed.

The particle measuring device (10) according to the present aspect may further include a light guiding lens (43, 201, 202, 203) configured to guide the light condensed by the condenser lens (42), to a light-entry-side end surface (50a, 210a, 220a, 230a) of the light transmitter (50, 210, 220, 230). With this configuration, even when displacement of the optical fibers has occurred, or even when the condensing position by the condenser lens has varied, the light condensed by the condenser lens can be guided to the light-entry-side end surface of the light transmitter. Thus, the state where the light appropriately enters the optical fibers can be maintained.

In this case, the light guiding lens (43, 201, 202, 203) may enlarge a diameter of the light condensed by the condenser lens (42), and may guide the light to the light-entry-side end surface (50a, 210a, 220a, 230a) of the light transmitter (50, 210, 220, 230). With this configuration, variation in the amount of light guided to the light detector in the subsequent stage by the light transmitter can be suppressed.

In the particle measuring device (10) according to the present aspect, the light guiding lens (43, 201, 202, 203) may cause the light condensed by the condenser lens (42) to enter, as a collimated light, the light-entry-side end surface (50a, 210a, 220a, 230a) of the light transmitter (50, 210, 220, 230). With this configuration, the light having entered the optical fibers of the light transmitter can each be inhibited from going out of the core without being reflected at the boundary between the core and the cladding. Thus, use efficiency of the light guided by the light transmitter can be increased.

The particle measuring device (10) according to the present aspect further includes an objective lens (41) configured to allow the light generated from the particle to pass. A distance between a rear-side principal plane of the objective lens (41) and a principal plane of the condenser lens (42), and a distance between the principal plane of the condenser lens (42) and a light-entry-side end surface (43a, 201a, 202a, 203a) of the light guiding lens (43, 201, 202, 203) may be each set to a focal length of the condenser lens (42). With this configuration, the objective lens, the condenser lens, and the light guiding lens form a telecentric optical system, and the light condensed by the condenser lens perpendicularly enters the light-entry-side end surface of the light guiding lens. Accordingly, the light having entered the light-entry-side end surface of the light guiding lens can be inhibited from leaking to the outside from the side face of the light guiding lens.

In the particle measuring device (10) according to the present aspect, the condenser lens (42) may converge the light generated from the particle, to a light-entry-side end surface (43a, 201a, 202a, 203a) of the light guiding lens (43, 201, 202, 203), and the light guiding lens (43, 201, 202, 203) may cause the light converged by the condenser lens (42) to enter the light-entry-side end surface (50a, 210a, 220a, 230a) of the light transmitter (50, 210, 220, 230), such that the light has an area having a diameter greater than that of a light entry end of each of the optical fibers (51, 211, 221, 231) and extending across a plurality of light entry ends of a plurality of the optical fibers (51, 211, 221, 231).

In the particle measuring device (10) according to the present aspect, a condition for a focal length of the light guiding lens (43, 201, 202, 203) can be represented by a formula below.

[Math 1]

$$f2 < \frac{\sqrt{m} \times b}{2 \times NA1}$$

In the formula above, f2 is the focal length of the light guiding lens, (43, 201, 202, 203), m is the number of the optical fibers (51, 211, 221, 231) of the light transmitter (50, 210, 220, 230), b is an outer diameter of each of the optical fibers (51, 211, 221, 231) in the light transmitter (50, 210, 220, 230), and NA1 is a numerical aperture of the condenser lens (42). With this configuration, the diameter of the luminous flux at the light-outputting-side end portion of the light guiding lens is smaller than the diameter of the light transmitter. Thus, the light having passed through the light guiding lens can be caused to assuredly enter the light transmitter.

In the particle measuring device (10) according to the present aspect, the light guiding lens (43, 201, 202, 203) may be a graded index lens in which a refractive index is reduced in accordance with increase in a distance from a central axis thereof.

In the particle measuring device (10) according to the present aspect, the light guiding lens (43, 201, 202, 203) may be adhered to light entry ends of a plurality of the optical fibers (51, 211, 221, 231).

In the particle measuring device (10) according to the present aspect, the light detector (300, 400, 500) may include: a light receiving element (331 to 336, 431 to 433, 531 to 536) configured to receive the light transmitted by the light transmitter (210, 220, 230); and an optical system disposed between the light transmitter (210, 220, 230) and the light receiving element (331 to 336, 431 to 433, 531 to 536) and configured to guide the light transmitted by the light transmitter (210, 220, 230), to the light receiving element (331 to 336, 431 to 433, 531 to 536).

In this case, the optical system may include a collimator lens (301, 401, 501) configured to convert lights outputted from a plurality of the optical fibers (211, 221, 231) of the light transmitter (210, 220, 230), into a collimated light. With this configuration, a space for disposing optical components can be easily provided between the collimator lens and the light receiving element.

In this case, the optical system may include a second condenser lens (321 to 326, 421 to 423, 521 to 526) disposed between the collimator lens (301, 401, 501) and the light receiving element (331 to 336, 431 to 433, 531 to 536). The second condenser lens (321 to 326, 421 to 423, 521 to 526) may condense the light converted into the collimated light by the collimator lens (301, 401, 501) and guide the light to the light receiving element (331 to 336, 431 to 433, 531 to 536). With this configuration, a space for disposing optical components can be easily provided between the collimator lens and the second condenser lens, and the light converted into the collimated light by the collimator lens can be assuredly guided to the light receiving element.

In the particle measuring device (10) according to the present aspect, the collimator lens (301, 401, 501) may be configured to be able to take in lights outputted from all of the optical fibers (211, 221, 231) forming the light transmitter (210, 220, 230), and the second condenser lens (321 to 326, 421 to 423, 521 to 526) may guide the lights taken in by the collimator lens (301, 401, 501), to the light receiving element (331 to 336, 431 to 433, 531 to 536). With this configuration, the amount of light guided to the light receiving element can be increased, and variation in the amount of light guided to the light receiving element can be suppressed, whereby the measurement accuracy can be increased.

In the particle measuring device (10) according to the present aspect, the light applicator (30) may apply, to the sample (11), a plurality of the irradiation lights having wavelengths different from each other. The particle measuring device (10) according to the present aspect may include: a plurality of the light transmitters (210, 220, 230) respectively corresponding to the plurality of the irradiation lights; and a plurality of the light detectors (300, 400, 500) configured to receive the lights respectively transmitted by the plurality of the light transmitters (210, 220, 230) and output detection signals. With this configuration, the lights generated from the particle due to the plurality of the irradiation lights are respectively guided to the light detectors via the light transmitters. Thus, the particle can be analyzed from various viewpoints in accordance with the amounts of lights received by the light detectors.

In this case, the plurality of the light detectors (300, 400, 500) may be set on base plates (300a, 400a, 500a) different from each other. With this configuration, when compared with a case where all of the light detectors are disposed on one base plate, the base plates can be freely arranged. Thus, the installation area of the device can be reduced. In addition, the lights are guided to the light detectors by the light transmitters each formed by optical fibers being bundled. Thus, when the light transmitters are disposed in a curved manner, the light detectors can be disposed at desired places.

In the particle measuring device (10) according to the present aspect, the light applicator (30) may apply the plurality of the irradiation lights to respective positions different from each other in a flow direction of the sample (11). With this configuration, it is easy to individually take out the lights respectively generated due to the plurality of the irradiation lights.

In the particle measuring device (10) according to the present aspect, the condenser lens (42) may inhibit chromatic aberration with respect to the lights respectively generated due to the plurality of the irradiation lights. With this configuration, a plurality of lights having different wavelengths can be condensed in a state where chromatic aberration is inhibited. Therefore, a plurality of lights can be accurately received at the light detector in a subsequent stage of the light transmitter.

In the particle measuring device (10) according to the present aspect, the sample (11) may include a cell as the particle, and a plurality of markers different from each other of the cell may be respectively stained by a plurality of fluorescent dyes that generate, due to the irradiation light, fluorescences having wavelengths different from each other. With this configuration, the cell can be analyzed from various viewpoints on the basis of the plurality of fluorescences generated from the single cell.

A second aspect of the present invention relates to a particle measuring method. The particle measuring method according to the present aspect includes: causing a sample (11) including a particle to flow in a flow cell (20); irradiating an irradiation light to the sample (11) flowing in the flow cell (20); condensing a light generated, by irradiation of the irradiation light, from the particle included in the sample (11); causing the condensed light to enter a light entry end of an optical fiber bundle formed by a plurality of optical fibers (51, 211, 221, 231) being bundled; and receiving the light transmitted by the optical fiber bundle and outputting a detection signal.

According to the particle measuring method of the present aspect, effects similar to those of the first aspect are exhibited.

In the particle measuring method according to the present aspect, the causing of the light to enter the light entry end of the optical fiber bundle includes enlarging a diameter of the condensed light and causing the resultant light to enter the light entry end of the optical fiber bundle.

ADVANTAGEOUS EFFECTS OF THE INVENTION

According to the present invention, the work for position adjustment of the optical fibers can be omitted, and a state where the light appropriately enters the optical fibers can be maintained.

BRIEF DESCRIPTION OF THE DRAWINGS

[FIG. 1] FIG. 1 is a schematic diagram showing a configuration of a particle measuring device according to Embodiment 1.
[FIG. 2] FIG. 2 is a schematic diagram showing a configuration of a particle measuring device according to Embodiment 2.
[FIG. 3] FIG. 3 is a block diagram showing a configuration of the particle measuring device according to Embodiment 2.
[FIG. 4] FIG. 4 is a schematic diagram showing a configuration of a light applicator according to Embodiment 2.
[FIG. 5] FIG. 5 is a schematic diagram showing configurations of a flow cell through light transmission parts according to Embodiment 2.
[FIG. 6A] FIG. 6A is a schematic diagram for describing converging action of light by a condenser lens according to Embodiment 2.
[FIG. 6B] FIG. 6B is a schematic diagram for describing converging action of light according to the condenser lens of Embodiment 2.
[FIG. 7A] FIG. 7A is a schematic diagram for describing transmission of light performed by the light transmission part according to Embodiment 2.
[FIG. 7B] FIG. 7B is a schematic diagram for describing transmission of light performed by the light transmission part according to Embodiment 2.
[FIG. 7C] FIG. 7C is a schematic diagram for describing transmission of light performed by the light transmission part according to Embodiment 2.
[FIG. 8] FIG. 8 is a schematic diagram showing a configuration of a light detector disposed in a subsequent stage of the light transmission part according to Embodiment 2.
[FIG. 9] FIG. 9 is a schematic diagram showing a configuration of a light detector disposed in the subsequent stage of the light transmission part according to Embodiment 2.
[FIG. 10] FIG. 10 is a schematic diagram showing a configuration of a light detector disposed in the subsequent stage of the light transmission part according to Embodiment 2.
[FIG. 11] FIG. 11 is a schematic diagram showing a state where base plates parallel to an X-Y plane are arranged in a Z-axis direction, according to Embodiment 2.

[FIG. 12] FIG. 12 is a schematic diagram for describing design and arrangement of a light detector disposed in the subsequent stage of the light transmission part according to Embodiment 2.

[FIG. 13] FIG. 13 is a schematic diagram for describing measurement and analysis performed by the particle measuring device according to Embodiment 2.

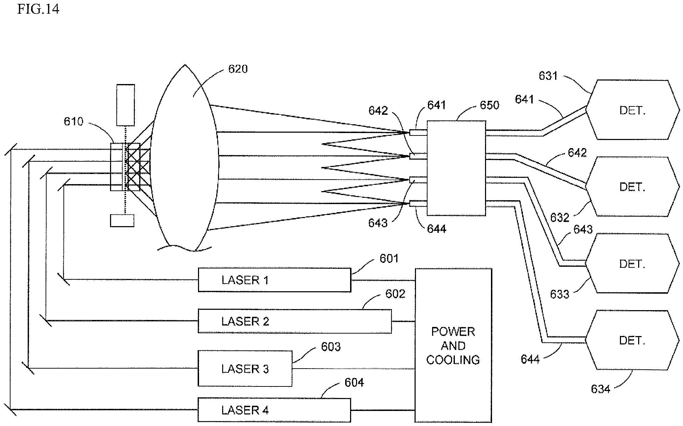

[FIG. 14] FIG. 14 is a schematic diagram for describing a configuration according to related art.

DESCRIPTION OF EMBODIMENTS

<Embodiment 1>

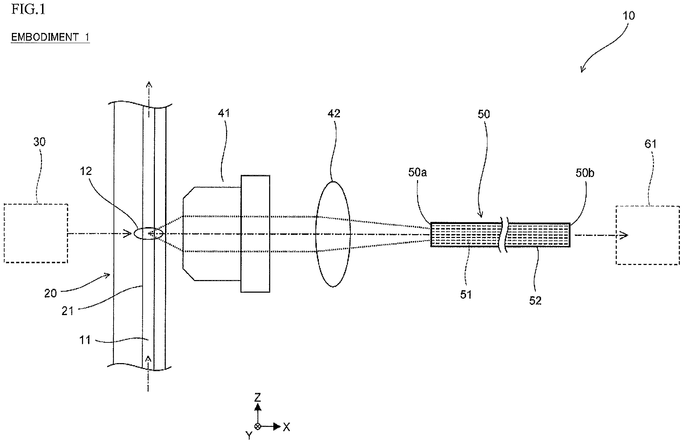

With reference to FIG. 1, a configuration of a particle measuring device 10 of Embodiment 1 is described. In FIG. 1, the XYZ axes are orthogonal to one another. The X-axis direction and the Y-axis direction correspond to directions parallel to the horizontal plane, and the Z-axis positive direction corresponds to the vertically upward direction. In the other drawings as well, the XYZ axes are set in the same manner as in FIG. 1.

The particle measuring device 10 includes a flow cell 20, a light applicator 30, an objective lens 41, a condenser lens 42, a light transmission part 50, and a light detector 61.

The flow cell 20 includes a flow path 21 extending in the Z-axis direction. A sample 11 including particles is caused to flow in the Z-axis positive direction in the flow path 21 of the flow cell 20. The light applicator 30 applies irradiation light to the sample 11 flowing in the flow cell 20. The irradiation light forms an elliptical beam spot 12 at a position of the flow path 21. The beam spot 12 indicates the region where the irradiation light is applied at the position of the flow path 21. When the irradiation light is applied to the sample 11, light is generated from a particle included in the sample 11. For convenience, FIG. 1 is shown such that the application direction of the irradiation light is in the X-axis positive direction. However, in actuality, the application direction of the irradiation light is the Y-axis positive direction.

The objective lens 41 condenses light generated, by application of the irradiation light, from a particle included in the sample 11. The condenser lens 42 condenses light having passed through the objective lens 41, and guides the condensed light to a light-entry-side end surface 50a of the light transmission part 50. The number of condenser lenses 42 to be used for guiding the light having passed through the objective lens 41 to the light transmission part 50 is not necessarily 1 as shown in FIG. 1, and a plurality of lenses may be used.

Here, the light generated from a particle could include a plurality of lights having different wavelengths. Thus, the condenser lens 42 is an achromatic lens for inhibiting chromatic aberration. Accordingly, a plurality of lights having different wavelengths can be condensed in a state where chromatic aberration is inhibited. Therefore, a plurality of lights having different wavelengths can be accurately detected at the light detector 61 provided in a subsequent stage of the light transmission part 50.

In a case where chromatic aberration due to a plurality of lights having different wavelengths does not pose a problem, or in a case where light generated from a particle includes only light having a single wavelength, the condenser lens 42 may not necessarily be an achromatic lens, and may be implemented as a general lens having a condensing action.

The light transmission part 50 is formed by a plurality of optical fibers 51 being bundled by a covering member 52. That is, an optical fiber bundle in which a plurality of optical fibers 51 are bundled is formed in the covering member 52. The light transmission part 50 is a so-called fiber bundle. Light condensed by the condenser lens 42 enters the light-entry-side end surface 50a of the light transmission part 50, i.e., a light entry end of the optical fiber bundle of the light transmission part 50. The condenser lens 42 causes the light to enter the light-entry-side end surface 50a of the light transmission part 50, such that the light has an area having a diameter greater than that of the light entry end of each optical fiber 51 at the light-entry-side end surface 50a and extending across a plurality of light entry ends of a plurality of optical fibers 51. The light having entered the light transmission part 50 is guided by the plurality of optical fibers 51 to a light-outputting-side end surface 50b of the light transmission part 50. The light detector 61 receives the light transmitted by the light transmission part 50, and outputs a detection signal.

Thus, according to the particle measuring device 10, the light condensed by the condenser lens 42 is guided to the light transmission part 50 formed by a plurality of optical fibers 51 being bundled. In this case, the diameter of the light-entry-side end surface 50a of the light transmission part 50 can be easily set to be greater than the diameter of the luminous flux condensed by the condenser lens 42. Accordingly, the light condensed by the condenser lens 42 can easily enter the light-entry-side end surface 50a of the light transmission part 50. Thus, even when a slight displacement of the optical fibers 51 has occurred, the light generated from the particle can be inhibited from being deviated from the light transmission part 50. Thus, the work of the user for position adjustment of the optical fibers 51 can be omitted, and the state where the light appropriately enters the optical fibers 51 can be maintained.

More specifically, light generated from the particle is condensed within the light-entry-side end surface 50a of the light transmission part 50. Accordingly, even when displacement of the optical fibers 51 has occurred, the state where the light appropriately enters the optical fibers 51 can be maintained.

The light generated from the particle is condensed at the light-entry-side end surface 50a of the light transmission part 50, such that the light has an area having a diameter greater than that of the light entry end of each optical fiber 51 and extending across a plurality of light entry ends of a plurality of optical fibers 51. Accordingly, when compared with a case where light generated from the particle enters the light-entry-side end surface 50a in a state where the light is narrowed to have a small area, the amount of light guided to the optical fibers 51 in the light transmission part 50 can be maintained at a constant level.

That is, light in a state of being narrowed to have a small area could enter only the cladding portion of an optical fiber 51 or a gap between a plurality of bundled optical fibers 51. In this case, the amount of light guided to the light detector 61 in the subsequent stage by the light transmission part 50 is significantly reduced. However, when the light condensed by the condenser lens 42 enters the light-entry-side end surface 50a such that the light has an area having a diameter greater than that of the light entry end of each optical fiber 51 and extending across light entry ends of a plurality of optical fibers 51, a part of the light enters the cladding portion or the gap, but the other part of the light enters the core portions of the optical fibers 51. Accordingly, variation in the amount of light guided to the light detector 61 in the subsequent stage by the light transmission part 50 can be suppressed.

In a general multi-color flow cytometry, for example, a plurality of fluorescences generated from a large number of target substances in one particle are simultaneously detected. In this case, in order to accurately detect the fluorescences, it is necessary to perform position adjustment of optical fibers every time a measurement is performed. However, if position adjustment is required for each measurement, work burden for the maintenance on a user is increased, and in addition, the user is required to have proficiency for performing the maintenance. According to the particle measuring device 10 of Embodiment 1, light generated from a particle is inhibited from being deviated from the optical fiber bundle in the light transmission part 50. Thus, the work burden on the user can be reduced, and high accuracy of the measurement result can be maintained.

<Embodiment 2>

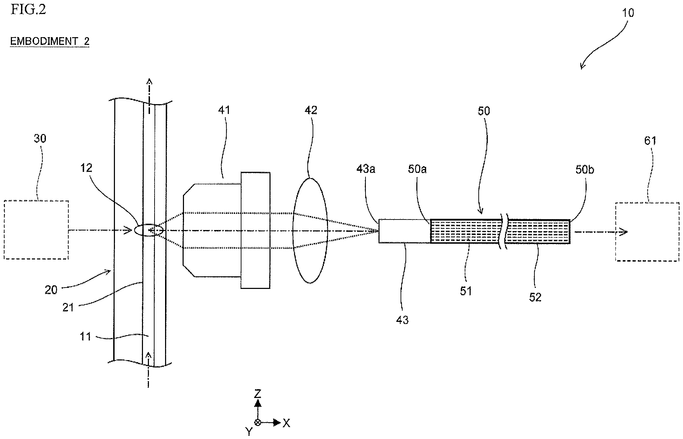

As shown in FIG. 2, the particle measuring device 10 of Embodiment 2 includes a light guiding lens 43 between the condenser lens 42 and the light transmission part 50, when compared with the particle measuring device 10 of Embodiment 1 shown in FIG. 1. The other configurations of Embodiment 2 are the same as those of Embodiment 1.

The light guiding lens 43 is a graded index lens in which the refractive index is reduced in accordance with increase in the distance from the central axis thereof. For example, the light guiding lens 43 is a SELFOC (registered trade mark) lens. The light guiding lens 43 may be a GRIN lens or a rod lens. In the light guiding lens 43, the refractive index is highest at the central axis extending in the X-axis direction, and the refractive index is reduced in accordance with increase in the distance from the central axis. The diameter of the luminous flux having entered the light guiding lens 43 changes, in the light guiding lens 43, at a cycle of a pitch length p in the advancing direction, and becomes minimum at every length p/2. The light guiding lens 43 is configured such that the length in the X-axis direction is p/4. Accordingly, the light condensed at a light-entry-side end surface 43a of the light guiding lens 43 becomes a collimated light in a state of being most expanded at the light-outputting-side end surface of the light guiding lens 43.

The condenser lens 42 causes light generated at the position of the beam spot 12, to be converged at the light-entry-side end surface 43a of the light guiding lens 43. The light guiding lens 43 guides, to the light-entry-side end surface 50a of the light transmission part 50, the light converged at the light-entry-side end surface 43a by the condenser lens 42. Accordingly, even when displacement of the optical fibers 51 has occurred, or even when the condensing position by the condenser lens 42 has varied, the light condensed by the condenser lens 42 can be guided to the light-entry-side end surface 50a of the light transmission part 50, as in Embodiment 1. Thus, the state where the light appropriately enters the optical fibers 51 can be maintained.

The condenser lens 42 enlarges the diameter of the light condensed by the condenser lens 42, and guides the resultant light to the light-entry-side end surface 50a of the light transmission part 50, i.e., the light entry end of the optical fiber bundle of the light transmission part 50. Accordingly, even when the position of the luminous flux entering the light-entry-side end surface 50a of the light transmission part 50 is displaced, variation in the amount of light guided to the light detector 61 in the subsequent stage by the light transmission part 50 can be suppressed, as in the case described with reference to FIG. 1.

<Specific configuration of Embodiment 2>

In the following, a specific configuration of Embodiment 2 is described. Among the components of the particle measuring device 10 described below, components having the same configurations as those shown in FIG. 2 are denoted by the same reference characters as those in FIG. 2. The components denoted by the same reference characters are not described, for convenience.

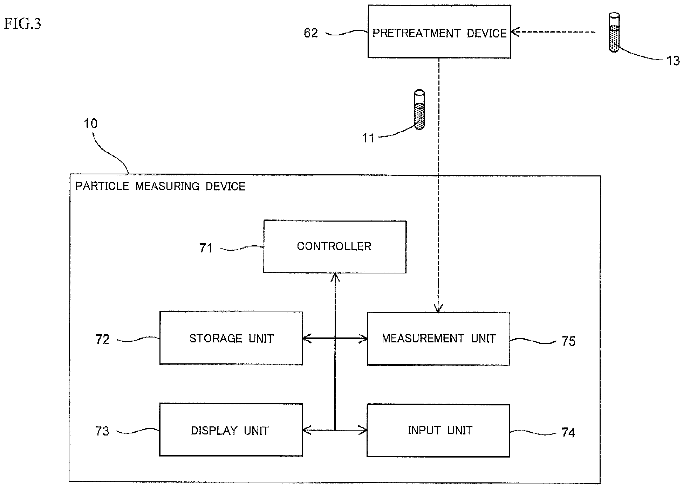

As shown in FIG. 3, the particle measuring device 10 measures a sample 11 prepared by a pretreatment device 62, and performs analysis thereof.

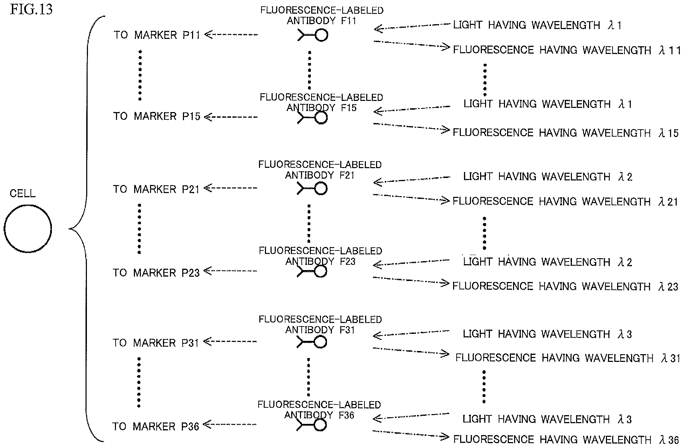

The pretreatment device 62 performs a process such as centrifugation on a specimen 13 of whole blood collected from a subject, and extracts hemocytes such as white blood cells, red blood cells, or platelets, as measurement target cells. The pretreatment device 62 includes: a mixing chamber for mixing a reagent and the specimen 13 having been subjected to a process such as centrifugation; a dispensing unit for dispensing the specimen 13 and the reagent into a mixing chamber; and the like. The pretreatment device 62 stains a plurality of markers different from each other of a measurement target cell, by a plurality of respective fluorescent dyes that generate, due to irradiation light, fluorescences having wavelengths different from each other. Thus, when the plurality of markers in a cell are stained by fluorescent dyes, the cell can be analyzed from various viewpoints on the basis of the plurality of fluorescences generated from the single cell. The cell analysis will be described later with reference to FIG. 13.

The specimen 13 is not limited to whole blood, and may be plasma, cerebrospinal fluid, tissue fluid, or urine. The measurement target cell is not limited to a hemocyte, and may be an epithelial cell, for example. The particles to be measured by the particle measuring device 10 are not limited to cells, and may be particles other than cells.

The particle measuring device 10 includes a controller 71, a storage unit 72, a display unit 73, an input unit 74, and a measurement unit 75. The controller 71 is a CPU. The controller 71 may be implemented by a CPU and a microcomputer. The controller 71 performs various processes on the basis of programs stored in the storage unit 72. The controller 71 is connected to the storage unit 72, the display unit 73, the input unit 74, and the measurement unit 75, receives signals from the units, and controls the units. The storage unit 72 is implemented by a RAM, a ROM, a hard disk, and the like. The display unit 73 is a liquid crystal display, for example. The input unit 74 is a mouse and a keyboard. The display unit 73 may be integrally formed with the input unit 74 as in a touch-panel-type display.

The measurement unit 75 measures the sample 11, receives light generated from each cell included in the sample 11, and outputs a detection signal based on the received light. The measurement unit 75 includes the flow cell 20, the light applicator 30, the objective lens 41, and the condenser lens 42 shown in FIG. 2, and in addition, light guiding lenses 201, 202, 203, light transmission parts 210, 220, 230, and light detectors 300, 400, 500 described later. The controller 71 performs analysis of each cell on the basis of the detection signal outputted from the measurement unit 75.

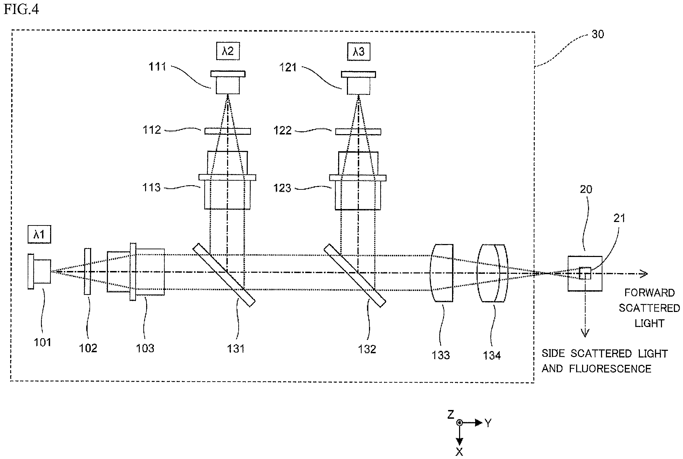

With reference to FIG. 4, a configuration of the light applicator 30 is described.

The light applicator 30 includes a light source 101, a filter 102, a collimator lens 103, a light source 111, a filter 112, a collimator lens 113, a light source 121, a filter 122, a collimator lens 123, dichroic mirrors 131, 132, a cylindrical lens 133, and a condenser lens 134.

The light sources 101, 111, 121 are semiconductor laser light sources. The light sources 101, 111, 121 emit lights having wavelengths $\lambda 1$, $\lambda 2$, $\lambda 3$ as the center wavelengths, respectively. Specifically, the wavelengths $\lambda 1$, $\lambda 2$, $\lambda 3$ are 488 nm, 642 nm, and 405 nm, respectively. The light sources 101, 111, 121 are not limited to semiconductor laser light sources, and may each be, for example, an optically pumped semiconductor laser light source, a solid-based laser light source, a laser light source based on gas such as He-Ne gas or Ar gas, or an LED light source.

Out of the light emitted from the light source 101, the filter 102 allows a light having the wavelength $\lambda 1$ to pass therethrough. The collimator lens 103 converts the light having passed through the filter 102, into a collimated light. Out of the light emitted from the light source 111, the filter 112 allows a light having the wavelength $\lambda 2$ to pass therethrough. The collimator lens 113 converts the light having passed through the filter 112, into a collimated light. Out of the light emitted from light source 121, the filter 122 allows a light having the wavelength $\lambda 3$ to pass therethrough. The collimator lens 123 converts the light having passed through the filter 122 into a collimated light.

The dichroic mirror 131 allows passage therethrough of the light having the wavelength $\lambda 1$ from the collimator lens 103, and reflects the light having the wavelength $\lambda 2$ from the collimator lens 113. The dichroic mirror 132 allows passage therethrough of the lights having the wavelengths $\lambda 1$, $\lambda 2$ from the dichroic mirror 131, and reflects the light having the wavelength $\lambda 3$ from the collimator lens 123. The dichroic mirrors 131, 132 are disposed such that the lights having the wavelengths $\lambda 1$, $\lambda 2$, $\lambda 3$ to be applied to the flow path 21 of the flow cell 20 are arranged at a predetermined interval in the Z-axis direction.

The cylindrical lens 133 converges the lights from the dichroic mirror 132, only in the X-axis direction. The condenser lens 134 converges the lights from the cylindrical lens 133 in the Z-axis direction, to be focused at the position of the flow path 21 of the flow cell 20. In addition, the condenser lens 134 converges the lights from the cylindrical lens 133 in the X-axis direction, to be focused at a position on the Y-axis negative side of the flow path 21. Accordingly, the lights condensed by the condenser lens 134 are applied, as irradiation lights, in a beam shape elongated in the X-axis direction, to the flow path 21.

When the irradiation lights are applied to the flow path 21 of the flow cell 20, the irradiation lights are applied to the sample 11 flowing in the Z-axis positive direction in the flow path 21. Accordingly, forward scattered light, side scattered light, and fluorescence are generated from each cell included in the sample 11. The forward scattered light is generated on the Y-axis positive side of the flow path 21, and the side scattered light and the fluorescence are generated around the flow path 21. A light receiving element disposed on the subsequent stage side of the flow cell 20 receives the side scattered light and the fluorescence generated on the X-axis positive side of the flow path 21. The light receiving element may receive the forward scattered light generated on the Y-axis positive side of the flow path 21.

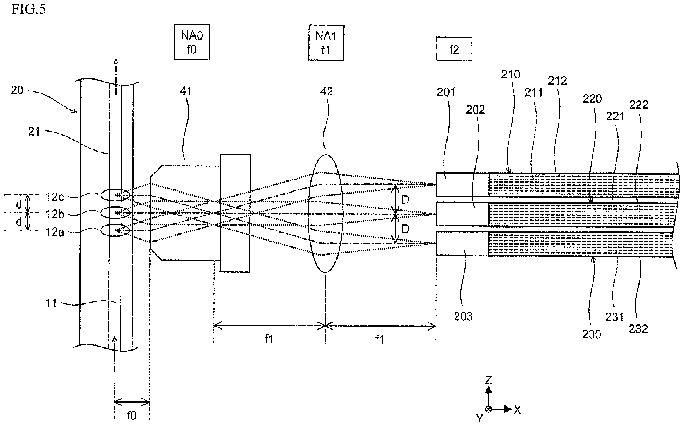

With reference to FIG. 5, configurations of the flow cell 20 through the light transmission parts 210, 220, 230 are described.

In the flow path 21 of the flow cell 20, three beam spots are formed by irradiation lights having the wavelengths $\lambda 1, \lambda 2, \lambda 3$. Specifically, beam spots 12a, 12b, 12c are formed at a predetermined interval d in the Z-axis positive direction in the flow path 21. The beam spots 12a, 12b, 12c indicate regions where the irradiation lights having the wavelengths $\lambda 1, \lambda 2, \lambda 3$, are applied, respectively. Thus, when the irradiation lights are applied to positions that are different from each other in the flow direction of the sample 11 in this manner, it is easy to individually take out the lights respectively generated due to the irradiation lights having the wavelengths $\lambda 1, \lambda 2, \lambda 3$.

The particle measuring device 10 includes the light transmission parts 210, 220, 230 and the light guiding lenses 201, 202, 203, which are arranged in the Z-axis negative direction. In addition, the particle measuring device 10 includes the light detectors 300, 400, 500 described later in the subsequent stages of the respective light transmission parts 210, 220, 230. The light detectors 300, 400, 500 correspond the light detector 61 shown in FIG. 2.

The light transmission parts 210, 220, 230 are configured similarly to the light transmission part 50 shown in FIG. 2. That is, the light transmission part 210 is formed by a plurality of optical fibers 211 being bundled by a covering member 212. The light transmission part 220 is formed by a plurality of optical fibers 221 being bundled by a covering member 222. The light transmission part 230 is formed by a plurality of optical fibers 231 being bundled by a covering member 232.

Similar to the light guiding lens 43 shown in FIG. 2, the light guiding lenses 201, 202, 203 are each a graded index lens in which the refractive index is reduced in accordance with increase in the distance from the central axis thereof. The light guiding lenses 201, 202, 203 are disposed at the light-entry-side end surfaces of the respective light transmission parts 210, 220, 230, using an adhesive. That is, the light guiding lens 201 is adhered to the light entry ends of the plurality of optical fibers 211 of the light transmission part 210. Similarly, the light guiding lens 202 is adhered to the light entry ends of the plurality of optical fibers 221 of the light transmission part 220. The light guiding lens 203 is adhered to the light entry ends of the plurality of optical fibers 231 of the light transmission part 230.

The light guiding lens 201 and the light transmission part 210 are adhered to each other such that the central axis of the light guiding lens 201 and the central axis of the light transmission part 210 are aligned with each other. Similarly, the light guiding lens 202 and the light transmission part 220 are adhered to each other such that the central axis of the light guiding lens 202 and the central axis of the light transmission part 220 are aligned with each other. The light guiding lens 203 and the light transmission part 230 are adhered to each other such that the central axis of the light guiding lens 203 and the central axis of the light transmission part 230 are aligned with each other. For the adhesion between the light guiding lenses 201, 202, 203, and the respective light transmission parts, an adhesive having a refractive index similar to that of the light guiding lenses 201, 202, 203 and the light transmission parts is used.

The light guiding lenses 201, 202, 203 and the light transmission parts 210, 220, 230 are disposed such that the central axis of the light guiding lens 201 and the light transmission part 210, the central axis of the light guiding lens 202 and the light transmission part 220, and the central axis of the light guiding lens 203 and the light transmission part 230 are arranged at a predetermined interval D in the Z-axis direction. The light-entry-side end surfaces of the light guiding lenses 201, 202, 203 are positioned at the same position in the X-axis direction.

When the irradiation lights having the wavelengths $\lambda 1, \lambda 2, \lambda 3$ are applied to the sample 11 flowing in the flow path 21, side scattered light and fluorescence are generated in the X-axis positive direction from a cell in the sample 11 at the positions of the beam spots 12a, 12b, 12c. The lights generated at the respective positions of the beam spots 12a, 12b, 12c are condensed by the objective lens 41 and the condenser lens 42, and enter the light guiding lenses 201, 202, 203, respectively. The condenser lens 42 is a lens that inhibits chromatic aberration as described above. That is, a plurality of lights having different wavelengths included in the light generated at the position of the beam spot 12a are converged at the light-entry-side end surface of the light guiding lens 201. A plurality of lights having different wavelengths included in the light generated at the position of the beam spot 12b are converged at the light-entry-side end surface of the light guiding lens 202. A plurality of lights having different wavelengths included in the light generated at the position of the beam spot 12c are converged at the light-entry-side end surface of the light guiding lens 203.

In this manner, the light guiding lens 201 is disposed between the condenser lens 42 and the light transmission part 210, the light guiding lens 202 is disposed between the condenser lens 42 and the light transmission part 220, and the light guiding lens 203 is disposed between the condenser lens 42 and the light transmission part 230. Accordingly, the lights condensed by the condenser lens 42 can be caused to smoothly enter the light-entry-side end surfaces of the light transmission parts 210, 220, 230.

Here, the distance between the rear-side principal plane of the objective lens 41 and the principal plane of the condenser lens 42 in the X-axis direction, and the distance between the principal plane of the condenser lens 42 and the light-entry-side end surfaces of the light guiding lenses 201, 202, 203 in the X-axis direction are each set to a focal length f1 of the condenser lens 42. Accordingly, the objective lens 41 and the condenser lens 42 form a telecentric optical system. Thus, the lights condensed by the condenser lens 42 perpendicularly enter the light-entry-side end surfaces of the light guiding lenses 201, 202, 203. When the lights perpendicularly enter the light-entry-side end surfaces of the light guiding lenses 201, 202, 203, the lights having entered the light guiding lenses 201, 202, 203 can be inhibited from leaking out from the side faces of the respective lenses.

When the light guiding lenses 201, 202, 203 are configured as described above and the lights perpendicularly enter the light-entry-side end surfaces of the light guiding lenses 201, 202, 203, the lights having entered the light guiding lenses 201, 202, 203 are converted into collimated lights by the light guiding lenses 201, 202, 203 to be guided to the light transmission parts 210, 220, 230, respectively. When the lights entering the light transmission parts 210, 220, 230 are collimated lights, the lights having entered the optical fibers 211, 221, 231 can each be inhibited from going out of the core without being reflected at the boundary between the core and the cladding. Therefore, lights leaking from the light transmission parts 210, 220, 230 can be suppressed, and thus, use efficiency of the lights guided by the light transmission parts 210, 220, 230 can be increased.

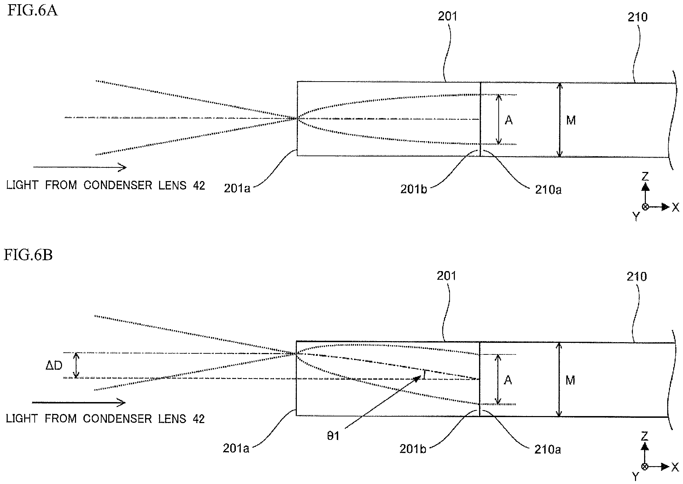

With reference to FIG. 6A and FIG. 6B, converging actions of light by the light guiding lenses 201, 202, 203 is described. Since the converging actions of light by the light guiding lenses 201, 202, 203 are the same with each other, only the action by the light guiding lens 201 is described here.

FIG. 6A shows a state where the positions of the light guiding lens 201 and the light transmission part 210 are appropriate. The condenser lens 42 converges the light having passed through the condenser lens 42, at the position of a light-entry-side end surface 201a of the light guiding lens 201, and causes the resultant light to perpendicularly enter the light-entry-side end surface 201a of the light guiding lens 201. The optical axis of the light entering the light-entry-side end surface 201a of the light guiding lens 201 is aligned with the central axis of the light guiding lens 201.

Due to the refracting action of the light guiding lens 201, the light from the condenser lens 42 becomes a collimated light having a diameter A, at a light-outputting-side end surface 201b of the light guiding lens 201 and a light-entry-side end surface 210a of the light transmission part 210. The diameter A of the luminous flux and the diameter of each optical fiber 211 of the light transmission part 210 are set such that the light entering the light-entry-side end surface 210a of the light transmission part 210 extends across a plurality of optical fibers 211 at the light-entry-side end surface 210a. That is, the light guiding lens 201 causes the light converged by the condenser lens 42 to enter the light-entry-side end surface 210a of the light transmission part 210, such that the light has an area having a diameter greater than that of the light entry end of each optical fiber 211 and extending across a plurality of light entry ends of a plurality of optical fibers 211.

FIG. 6B shows a state where the position of the light guiding lens 201 and the light transmission part 210 is displaced by ΔD in the Z-axis negative direction. In this case, the optical axis of the light entering the light-entry-side end surface 201a of the light guiding lens 201 is displaced by ΔD from the central axis of the light guiding lens 201. In this case as well, due to the refracting action of the light guiding lens 201, the light from the condenser lens 42 becomes a collimated light having the diameter A at the light-outputting-side end surface 201b of the light guiding lens 201. The region, at the light-outputting-side end surface 201b, where the luminous flux passes is substantially the same as the region where the luminous flux passes in the case where there is no displacement shown in FIG. 6A.

Thus, even where the positions of the light guiding lens 201 and the light transmission part 210 are displaced, the luminous flux becomes a collimated light having the diameter A at the light-outputting-side end surface 201b of the light guiding lens 201, and the region, at the light-outputting-side end surface 201b, where the luminous flux passes does not substantially change. Therefore, even if a slight displacement of the light guiding lens 201 and the light transmission part 210 has occurred, the light from the condenser lens 42 is assuredly guided to the light-entry-side end surface 210a of the light transmission part 210, without being deviated from the light transmission part 210. Therefore, the state where the light appropriately enters the light transmission part 210 can be maintained.

As shown in FIG. 6B, when the light guiding lens 201 is provided at a previous stage of the light transmission part 210, the allowance for displacement of the light guiding lens 201 and the light transmission part 210 is increased when compared with a case where the light from the condenser lens 42 directly enters the light transmission part 210 as shown in FIG. 1. For example, in a case where the light from the condenser lens 42 directly enters the light transmission part 210 in the form of luminous flux having the diameter A, if the diameter of the light transmission part 210 is defined as M, the allowance for displacement is (M-A)/2. Meanwhile, in the case of the configuration shown in FIG. 6B, since the light from the condenser lens 42 only needs to enter the light-entry-side end surface 201a of the light guiding lens 201, the allowance for displacement is M/2. Therefore, in the case of the configuration shown in FIG. 6B, the allowance for displacement is increased when compared with the case where the light from the condenser lens 42 directly enters the light transmission part 210.

Even when the position of the beam spot 12a has been displaced, the light from the condenser lens 42 enters, similarly to the case shown in FIG. 6B, at a position displaced from the central axis of the light guiding lens 201 and the light transmission part 210 in the light-entry-side end surface 201a of the light guiding lens 201. Therefore, even when the position of the beam spot 12a has been displaced, similarly, the light from the condenser lens 42 is assuredly guided to the light transmission part 210, without being deviated from the light transmission part 210.

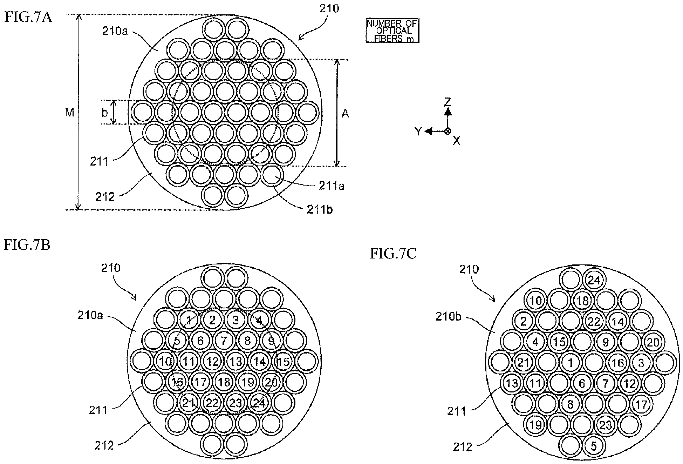

With reference to FIG. 7A to FIG. 7C, transmissions of light by the light transmission parts 210, 220, 230 are described. Since transmissions of light by the light transmission parts 210, 220, 230 are the same with each other, only the transmission of light by the light transmission part 210 is described.

As shown in FIG. 7A, at the light-entry-side end surface 210a of the light transmission part 210, a plurality of optical fibers 211 are bundled by the covering member 212. Each optical fiber 211 includes a core 211a and a cladding 211b. The core 211a is covered by the cladding 211b.

In FIG. 7A, the luminous flux entering the light-entry-side end surface 210a from the light guiding lens 201 is indicated by a dotted-line circle. When the optical fibers 211 through which the luminous flux passes at the light-entry-side end surface 210a of the light transmission part 210 are numbered as 1 to 24 as shown in FIG. 7B, the optical fibers 211 numbered as 1 to 24 are distributed as shown in FIG. 7C, for example, at a light-outputting-side end surface 210b of the light transmission part 210. The reason why the optical fibers 211 numbered as 1 to 24 are distributed at random at the light-outputting-side end surface 210b in this manner is that the light transmission part 210 is produced by the optical fibers 211 being bundled at random. Therefore, it is difficult to: specify the optical fibers 211 through which the luminous flux has passed at the light-entry-side end surface 210a; and select corresponding optical fibers 211 at the light-outputting-side end surface 210b, to guide the light to the light detector in the subsequent stage of the light transmission part 210.

Therefore, lights outputted from all of the optical fibers 211 forming the light transmission part 210 are guided to the light detector 300 disposed in the subsequent stage of the light transmission part 210. Similarly, lights outputted from all of the optical fibers 221 forming the light transmission part 220 are guided to the light detector 400 disposed in the subsequent stage of the light transmission part 220, and lights outputted from all of the optical fibers 231 forming the light transmission part 230 are guided to the light detector 500 disposed in the subsequent stage of the light transmission part 230. Specifically, collimator lenses disposed in the subsequent stages of the light transmission parts are each configured to be able to take in lights outputted from all of the optical fibers forming the corresponding light transmission part. Accordingly, the amount of light guided to a light receiving element disposed in the light detector can be increased, and variation in the amount of light guided to the light receiving element can be suppressed, whereby the measurement accuracy can be increased.

Here, designs and arrangement of optical components of the flow cell 20 through the light transmission parts 210, 220, 230 are described.

As shown in FIG. 5, the numerical aperture of the objective lens 41 is defined as NA0, the focal length of the objective lens 41 is defined as f0, the numerical aperture of the condenser lens 42 is defined as NA1, the focal length of the condenser lens 42 is defined as f1, and the focal length of the light guiding lenses 201, 202, 203 is defined as f2. As described above, the interval between the beam spots 12a, 12b, 12c is defined as d, and the interval between the central axes of the light guiding lenses 201, 202, 203 is D. As shown in FIG. 6A and FIG. 6B, the diameter of the luminous flux entering the light transmission part 210, 220, 230 is defined as A, and the diameter of the light transmission part 210, 220, 230 is defined as M. In addition, as shown in FIG. 7A, the number of optical fibers in the light transmission part 210, 220, 230 is defined as m, and the outer diameter of each optical fiber is defined as b.

A lateral magnification $\beta 1$ based on the objective lens 41 and the condenser lens 42 is represented by formula (1) below.

$$\beta 1 = NA0/NA1 = f1/f0 = D/d \quad \cdots (1)$$

The interval D is preferably greater than 1 mm from the viewpoint of ease of manufacturing the light transmission parts 210, 220, 230. In order that lights generated at the positions of the beam spots 12a, 12b, 12c are appropriately associated with each other as lights generated from a single cell, the interval d is preferably smaller than 0.2 mm. Therefore, when the conditions of the intervals D, d are added to formula (1) above, it can be said that $\beta 1 > 5$ is preferable.

The numerical aperture NA0 of the objective lens 41 is preferably not smaller than 0.7 in order to efficiently condense the lights generated from the cell included in the sample 11. The objective lens 41 is provided at a position separated by the focal length f0 from the flow path 21. As described above, the interval between the objective lens 41 and the condenser lens 42, i.e., the interval between the rear-side principal plane of the objective lens 41 and the condenser lens 42, is set to the focal length f1 of the condenser lens 42. The interval between the condenser lens 42 and the light guiding lenses 201, 202, 203 is set to the focal length f1 of the condenser lens 42.

The diameter A of the luminous flux entering the light transmission part 210, 220, 230 is represented by formula (2) below.

$$A = 2 \times f2 \times NA1 \quad \cdots (2)$$

The diameter M of the light transmission part 210, 220, 230 is represented by formula (3) below.

[Math 2]

$$M = \sqrt{m} \times b \quad \cdots (3)$$

The diameter A of the luminous flux entering the light transmission part 210, 220, 230 needs to be smaller than the diameter M of the light transmission part 210, 220, 230. Thus, the relationship between A and M is A<M. Thus, when A and M of formulae (2) and (3) above are assigned the condition of A<M, the condition of the focal length f2 of the light guiding lens 201, 202, 203 is represented by formula (4) below.

[Math 3]

$$f2 < \frac{\sqrt{m} \times b}{2 \times NA1} \quad \cdots (4)$$

When the condition of formula (4) above is satisfied, the lights having passed through the light guiding lenses 201, 202, 203 can be caused to assuredly enter the respective light transmission parts 210, 220, 230.

In order that each luminous flux enters the corresponding light transmission part 210, 220, 230 such that the luminous flux extends across a plurality of optical fibers in the light transmission part 210, 220, 230, the number m of optical fibers in the light transmission part 210, 220, 230 is preferably greater than 4. In addition, the diameter M of the light transmission part is preferably greater than the diameter of the condenser lens such that, even when an error in an accuracy range has occurred in the adhesion between the condenser lens and the light transmission part, all of the lights outputted from the light-outputting-side end surface of the condenser lens enters the light transmission part.

When the beam spot 12a has been displaced by $\Delta d$ in the Z-axis negative direction, the light condensed by the condenser lens 42 is displaced by $\Delta D$ in the Z-axis positive direction at the light-entry-side end surface 201a of the light guiding lens 201, as shown in FIG. 6B. The displaced amount $\Delta D$ is expressed as $\Delta D = \beta 1 \times \Delta d$. Therefore, the lateral magnification $\beta 1$ and the diameter of the light-entry-side end surface 201a of the light guiding lens 201 are preferably set in accordance with an expected displaced amount $\Delta d$ of the beam spot 12a.

The entry position of the light from the condenser lens 42 changes at the light-entry-side end surface 201a of the light guiding lens 201 in accordance with displacement of the light guiding lens 201 and displacement of the beam spot 12a. As shown in FIG. 6B, in a case where the displacement at the light-entry-side end surface 201a is $\Delta D$, the optical axis of the light entering the light-entry-side end surface 210a of the light transmission part 210 is inclined by an angle $\theta 1$ relative to the X-axis direction. When the angle $\theta 1$ is too large, the light having entered optical fibers 211 of the light transmission part 210 go out of the cores 211a without being reflected at the boundaries between the cores 211a and the claddings 211b. Therefore, the angle $\theta 1$ is preferably small to an extent that allows the light having entered the optical fibers 211 to advance in the cores 211a.

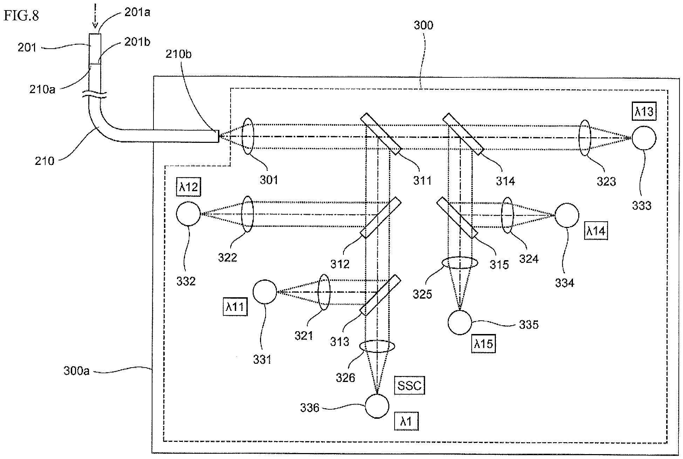

With reference to FIG. 8, a configuration of the light detector 300 disposed in the subsequent stage of the light transmission part 210 is described.

The light having entered the light-entry-side end surface 201a of the light guiding lens 201 passes within the light guiding lens 201, and is guided to the light-outputting-side end surface 201b of the light guiding lens 201. The light-entry-side end surface 210a of the light transmission part 210 is adhered to the light-outputting-side end surface 201b of the light guiding lens 201. The light-outputting-side end surface 210b of the light transmission part 210 is set at a base plate 300a. The light detector 300 is set on the base plate 300a. The light detector 300 includes a collimator lens 301, dichroic mirrors 311, 312, 313, 314, 315, second condenser lenses 321, 322, 323, 324, 325, 326, and light receiving elements 331, 332, 333, 334, 335, 336.

The collimator lens 301 converts the light outputted from the light-outputting-side end surface 210b of the light transmission part 210 into a collimated light. The light from the light-outputting-side end surface 210b includes fluorescences of which the center wavelengths are wavelengths $\lambda 11$, $\lambda 12$, $\lambda 13$, $\lambda 14$, $\lambda 15$, and a side scattered light of which the center wavelength is the wavelength $\lambda 1$. The dichroic mirror 311 reflects the fluorescences having the wavelengths $\lambda 11$, $\lambda 12$ and the side scattered light having the wavelength $\lambda 1$, and allows passage therethrough of the fluorescences having the wavelengths $\lambda 13$, $\lambda 14$, $\lambda 15$. The dichroic mirror 312 reflects the fluorescence having the wavelength $\lambda 12$, and allows passage therethrough of the fluorescence having the wavelength $\lambda 11$ and the side scattered light having the wavelength $\lambda 1$. The dichroic mirror 313 reflects the light having the wavelength $\lambda 11$ and allows passage therethrough of the side scattered light having the wavelength $\lambda 1$. The dichroic mirror 314 reflects the fluorescences having the wavelengths $\lambda 14$, $\lambda 15$ and allows passage therethrough of the fluorescence having the wavelength $\lambda 13$. The dichroic mirror 315 reflects the fluorescence having the wavelength $\lambda 14$ and allows passage therethrough of the fluorescence having the wavelength λ15.

The second condenser lenses 321, 322, 323, 324, 325 converge the fluorescences having the wavelengths λ11, λ12, λ13, λ14, λ15, respectively. The second condenser lens 326 converges the side scattered light having the wavelength λ1. The light receiving elements 331 to 335 receive fluorescences converged by the second condenser lenses 321 to 325, and output detection signals in accordance with the intensities of the received fluorescences, respectively. The light receiving element 336 receives the side scattered light converged by the second condenser lens 326, and outputs a detection signal in accordance with the intensity of the received side scattered light. The light receiving elements 331 to 336 are each a photomultiplier tube (PMT). Since the light receiving elements 331 to 336 are implemented as the photomultiplier tubes, the light receiving sensitivity can be increased.

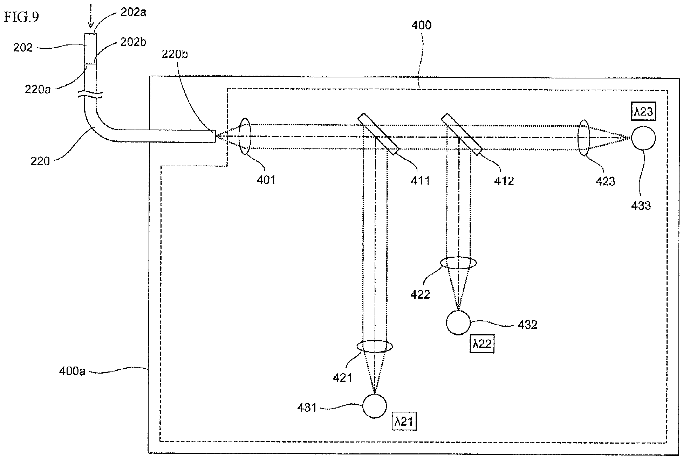

With reference to FIG. 9, a configuration of the light detector 400 disposed in the subsequent stage of the light transmission part 220 is described.

The light having entered a light-entry-side end surface 202a of the light guiding lens 202 passes within the light guiding lens 202, and is guided to a light-outputting-side end surface 202b of the light guiding lens 202. A light-entry-side end surface 220a of the light transmission part 220 is adhered to the light-outputting-side end surface 202b of the light guiding lens 202. A light-outputting-side end surface 220b of the light transmission part 220 is set at a base plate 400a. The light detector 400 is set on the base plate 400a. The light detector 400 includes a collimator lens 401, dichroic mirrors 411, 412, second condenser lenses 421, 422, 423, and light receiving elements 431, 432, 433.

The collimator lens 401 converts the light outputted from the light-outputting-side end surface 220b of the light transmission part 220 into a collimated light. The light from the light-outputting-side end surface 220b includes fluorescences of which the center wavelengths are wavelengths λ21, λ22, λ23. The dichroic mirror 411 reflects the fluorescence having the wavelength λ21 and allows passage therethrough of the fluorescences having the wavelengths λ22, λ23. The dichroic mirror 412 reflects the fluorescence having the wavelength λ22 and allows passage therethrough of the fluorescence having the wavelength λ23.

The second condenser lenses 421, 422, 423 converge the fluorescences having the wavelengths λ21, λ22, λ23, respectively. The light receiving elements 431 to 433 receive fluorescences converged by the second condenser lenses 421 to 423, and output detection signals in accordance with the intensities of the received fluorescences, respectively. The light receiving elements 431 to 433 are each a photomultiplier tube (PMT). Since the light receiving elements 431 to 433 are implemented as the photomultiplier tubes, the light receiving sensitivity can be increased.

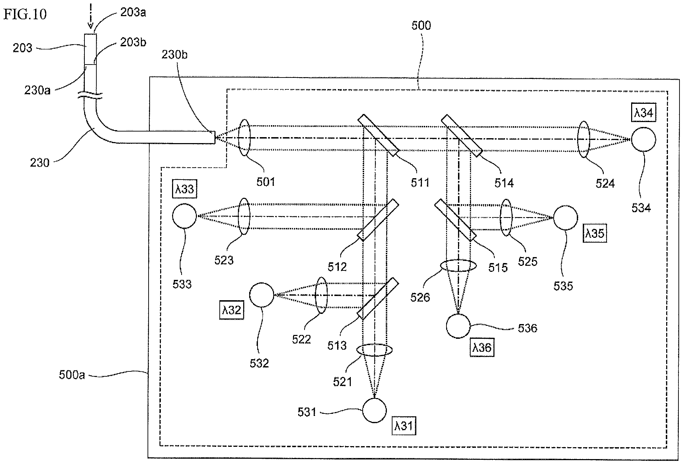

With reference to FIG. 10, a configuration of the light detector 500 disposed in the subsequent stage of the light transmission part 230 is described.

The light having entered a light-entry-side end surface 203a of the light guiding lens 203 passes within the light guiding lens 203, and is guided to a light-outputting-side end surface 203b of the light guiding lens 203. A light-entry-side end surface 230a of the light transmission part 230 is adhered to the light-outputting-side end surface 203b of the light guiding lens 203. A light-outputting-side end surface 230b of the light transmission part 230 is set at a base plate 500a. The light detector 500 is set on the base plate 500a. The light detector 500 includes a collimator lens 501, dichroic mirrors 511, 512, 513, 514, 515, second condenser lenses 521, 522, 523, 524, 525, 526, and light receiving elements 531, 532, 533, 534, 535, 536.

The collimator lens 501 converts the light outputted from the light-outputting-side end surface 230b of the light transmission part 230 into a collimated light. The light from the light-outputting-side end surface 230b includes fluorescences of which the center wavelengths are wavelengths λ31, λ32, λ33, λ34, λ35, λ36. The dichroic mirror 511 reflects fluorescences having the wavelengths λ31, λ32, λ33, and allows passage therethrough of fluorescences having the wavelengths λ34, λ35, λ36. The dichroic mirror 512 reflects the fluorescence having the wavelength λ33 and allows passage therethrough of the fluorescences having the wavelengths λ31, λ32. The dichroic mirror 513 reflects the light having the wavelength λ32 and allows passage therethrough of the fluorescence having the wavelength λ31. The dichroic mirror 514 reflects fluorescences having the wavelengths λ35, λ36, and allows passage therethrough of the fluorescence having the wavelength λ34. The dichroic mirror 515 reflects the fluorescence having the wavelength λ35 and allows passage therethrough of the fluorescence having the wavelength λ36.

The second condenser lenses 521, 522, 523, 524, 525, 526 converge the fluorescences having the wavelengths λ31, λ32, λ33, λ34, λ35, λ36, respectively. The light receiving elements 531 to 536 receive fluorescences converged by second condenser lenses 521 to 526, and output detection signals in accordance with the intensities of the received fluorescences, respectively. The light receiving elements 531 to 536 are each a photomultiplier tube (PMT). Since the light receiving elements 531 to 536 are implemented as the photomultiplier tubes, the light receiving sensitivity can be increased.

As shown in FIGS. 8 to 10, the light detectors 300, 400, 500 are set on the base plates 300a, 400a, 500a different from one another. This makes it easy to individually and freely set the angle of disposition of each base plate, such as disposing the base plate 300a, 400a, 500a perpendicularly to the installation plane of the particle measuring device 10, or disposing the base plate 300a, 400a, 500a in parallel to the installation plane of the particle measuring device 10. Therefore, when compared with a case where all of the light detectors 300, 400, 500 are disposed on one base plate, the base plates 300a, 400a, 500a can be freely arranged. Thus, the installation area of the particle measuring device 10 can be reduced.

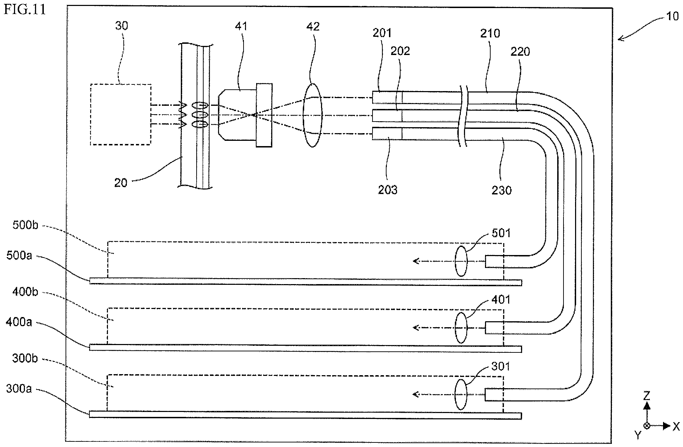

When each base plate is disposed in parallel to the installation plane of the particle measuring device 10, for example, the base plates 300a, 400a, 500a in parallel to the X-Y plane are disposed so as to be arranged in the Z-axis direction, as shown in FIG. 11. In this case, the light detector 300 is disposed in a region 300b above the base plate 300a, the light detector 400 is disposed in a region 400b above the base plate 400a, and the light detector 500 is disposed in a region 500b above the base plate 500a. For convenience, in FIG. 11, only the collimator lenses 301, 401, 501 among the components of the respective light detectors are shown. When each base plate is disposed as shown in FIG. 11, the installation area, i.e., the area in the X-Y plane, of the particle measuring device 10, can be reduced.

When all of the light detectors 300, 400, 500 are disposed on a single base plate, the size of the base plate is increased, and thus, the base plate is easily deflected. In this case, in accordance with the deflection of the base plate, displacement occurs between the light detectors. However, when the light detectors 300, 400, 500 are respectively disposed on the base plates 300a, 400a, 500a which are different from each other, the size of the base plate 300a, 400a, 500a can be reduced. Therefore, deflection of the base plates are suppressed when compared with a case where a single base plate is used, and thus, displacement that could occur between the light detectors can be prevented in advance.

The lights from the condenser lens 42 are guided to the light detectors 300, 400, 500 via the light transmission parts 210, 220, 230, respectively. In this case, since the light transmission parts 210, 220, 230 are each formed by optical fibers being bundled, the light transmission parts 210, 220, 230 need not necessarily be linearly disposed. Therefore, when the light transmission parts 210, 220, 230 are disposed in a curved manner, the light detectors 300, 400, 500 can be disposed at desired places.

The lights generated due to the irradiation lights having a plurality of the wavelengths $\lambda 1$, $\lambda 2$, $\lambda 3$ are guided to the light detectors 300, 400, 500 by the light transmission parts 210, 220, 230, respectively. Accordingly, the cell can be analyzed from various viewpoints in accordance with the amounts of lights received by the light receiving elements of the light detectors 300, 400, 500.

In FIGS. 8 to 10, the light receiving elements 331 to 336, 431 to 433, 531 to 536 may each be implemented as an avalanche photodiode (APD) or a photodiode (PD). Instead of each of the dichroic mirrors 311 to 315, a half mirror may be provided. In this case, with respect to the lights having been reflected by the half mirror and having passed through the half mirror, only a light having a desired wavelength is guided to a light receiving element by a filter provided in a subsequent stage of the half mirror. Similarly, instead of each of the dichroic mirrors 411, 412, 511 to 515, a half mirror may be provided, and a filter for guiding only a light having a desired wavelength to a light receiving element may be provided in a subsequent stage of the half mirror.

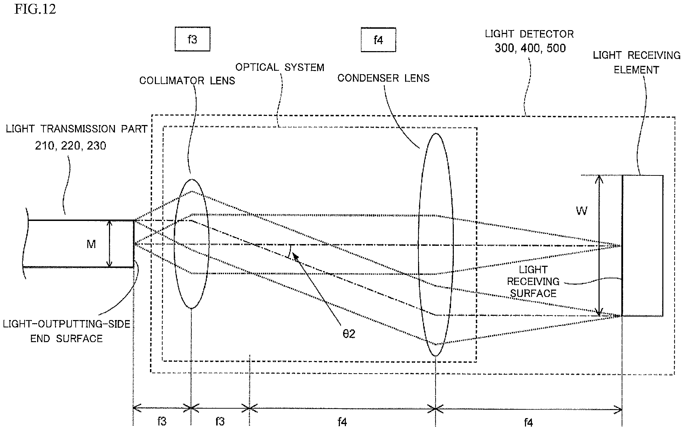

With reference to FIG. 12, designs and arrangement of the light detectors 300, 400, 500 respectively disposed in the subsequent stages of the light transmission parts 210, 220, 230 are described. For convenience, in FIG. 12, one light transmission part, one light detector, one collimator lens, one condenser lens, and one light receiving element only are shown as representatives.

That is, in the light transmission part 210 and the light detector 300, the collimator lens shown in FIG. 12 corresponds to the collimator lens 301, the condenser lens shown in FIG. 12 corresponds to the second condenser lenses 321 to 326, and the light receiving element shown in FIG. 12 corresponds to the light receiving elements 331 to 336. In the light transmission part 220 and the light detector 400, the collimator lens shown in FIG. 12 corresponds to the collimator lens 401, the condenser lens shown in FIG. 12 corresponds to the second condenser lenses 421 to 423, and the light receiving element shown in FIG. 12 corresponds to the light receiving elements 431 to 433. In the light transmission part 230 and the light detector 500, the collimator lens shown in FIG. 12 corresponds to the collimator lens 501, the condenser lens shown in FIG. 12 corresponds to the second condenser lenses 521 to 526, and the light receiving element shown in FIG. 12 corresponds to the light receiving elements 531 to 536. The collimator lens, the second condenser lens, and the dichroic mirror disposed between the light transmission part and the light receiving element correspond to an optical system described in the claims.

As shown in FIG. 12, the diameter of the light transmission part is defined as M, the focal length of the collimator lens is defined as f3, the focal length of the condenser lens is defined as f4, and the diameter of the light receiving surface of the light receiving element is defined as W.

The collimator lens is disposed at a position separated by the focal length f3 of the collimator lens from the light-outputting-side end surface of the light transmission part. The condenser lens is disposed at a position separated by the focal length f4 of the condenser lens from the light receiving surface of the light receiving element. Then, the optical path length between the collimator lens and the condenser lens, more specifically, the optical path length of the light that passes the center of the collimator lens and the center of the condenser lens, is set to be f3+f4. When the collimator lens and the condenser lens are disposed in this manner, the collimator lens and the condenser lens form a telecentric optical system, and the light condensed by the condenser lens perpendicularly enters the light receiving surface of the light receiving element.

If a case where a light outputted from an outermost position of the light-outputting-side end surface of the light transmission part enters at an outermost position of the light receiving surface of the light receiving element is taken into consideration, a lateral magnification β2 based on the collimator lens and the condenser lens is represented by formula (5) below.

$$\beta2 = f4/f3 = W/M \quad \cdots (5)$$

Therefore, for example, in a case where the diameter M of the light transmission part and the diameter W of the light receiving surface of the light receiving element are determined in advance, the lateral magnification β2 is determined on the basis of formula (5) above. Then, the values of the focal lengths f3, f4 are determined such that the ratio of the focal lengths f3, f4 becomes the lateral magnification β2. Then, the optical path length between the collimator lens and the condenser lens is determined to be f3+f4. Alternatively, in accordance with the focal lengths f3, f4, the lateral magnification β2 may be determined, and the values of the diameters M, W may be determined.

When a light is outputted from an outermost position of the light-outputting-side end surface of the light transmission part, the optical axis of this light is inclined by an angle θ2 relative to the optical axis of a case where a light is outputted from the center of the light-outputting-side end surface of the light transmission part. When the angle θ2 is too large in this case, the light cannot be appropriately reflected by the dichroic mirror disposed between the collimator lens and the condenser lens. Therefore, the angle θ2 is preferably small to an extent that allows the light can be appropriately reflected by the dichroic mirror.

As described above, the collimator lens converts the light outputted from the light transmission part into a collimated light. Accordingly, a space for disposing optical components can be easily provided between the collimator lens and the light receiving element. The condenser lens condenses the light converted into the collimated light by the collimator lens, and guides the resultant light to the light receiving element. Accordingly, a space for disposing optical components can be easily provided between the collimator lens and the condenser lens, and the light converted into the collimated light by the collimator lens can be assuredly guided to the light receiving element.

The condenser lens may be omitted. In this case, the light receiving element receives the light converged by the collimator lens. Alternatively, both of the collimator lens and the condenser lens may be omitted. In this case, the light receiving element receives the light outputted from the light-outputting-side end surface of the light transmission part.

Next, with reference to FIG. 13, measurement and analysis performed by the particle measuring device 10 are described.

The pretreatment device 62 fluorescently labels markers different from each other of a cell. At this time, in the case of Embodiment 2, the maximum number of markers fluorescently labeled for a single cell is 14. FIG. 13 shows an example in which, for a single cell, the pretreatment device 62 fluorescently labels 14 markers of P11 to P15, P21 to P23, P31 to P36, which are different from each other. These markers are, for example, antigens present on the surface or in the cytoplasm of the cell.

The pretreatment device 62 fluorescently labels the markers P11 to P15, P21 to P23, P31 to P36 of the cell, by using fluorescence-labeled antibodies F11 to F15, F21 to F23, F31 to F36, respectively. The fluorescence-labeled antibodies F11 to F15, F21 to F23, F31 to F36 include antibodies that bind to the markers P11 to P15, P21 to P23, P31 to P36 through antigen-antibody reaction, respectively. The fluorescence-labeled antibodies F11 to F15 include fluorescent dyes that generate, by being irradiated with a light having the wavelength λ1, fluorescences having the wavelengths λ11, λ12, λ13, λ14, λ15. The fluorescence-labeled antibodies F21 to F23 include fluorescent dyes that generate, by being irradiated with a light having the wavelength λ2, fluorescence having the wavelengths λ21, λ22, λ23. The fluorescence-labeled antibodies F31 to F36 include fluorescent dyes that generate, by being irradiated with a light having the wavelength λ3, fluorescences having the wavelengths λ31, λ32, λ33, λ34, λ35, λ36.

The sample 11 prepared by the pretreatment device 62 includes the cell fluorescently labeled by these fluorescence-labeled antibodies. When the sample 11 is caused to flow in the flow path 21 of the flow cell 20 and the lights having the wavelengths λ1, λ2, λ3 are applied, fluorescences having the wavelengths λ11, λ12, λ13, λ14, λ15 and a side scattered light having the wavelength λ1 are generated at the position of the beam spot 12a, fluorescences having the wavelengths λ21, λ22, λ23 are generated at the position of the beam spot 12b, and fluorescences having the wavelengths λ31, λ32, λ33, λ34, λ35, λ36 are generated at the position of the beam spot 12c.

With respect to a single cell, the lights generated at the positions of the beam spots 12a, 12b, 12c are received by predetermined light receiving elements in accordance with the wavelengths, as described with reference to FIGS. 8 to 10. Detection signals based on the fluorescences and side scattered light generated from the single cell are associated with one another. Cell analysis is performed on the basis of detection signals based on the fluorescences having the wavelengths λ11, λ12, λ13, λ14, λ15, a detection signal based on the side scattered light having the wavelength λ1, detection signals based on the fluorescences having the wavelengths λ21, λ22, λ23, and detection signals based on the fluorescences having the wavelengths λ31, λ32, λ33, λ34, λ35, λ36, which have been obtained from the single cell.

In the example shown in FIG. 13, with respect to a single one cell, 14 markers different from one another are fluorescently labeled. However, out of the markers of the cell, only necessary markers may be fluorescently labeled in accordance with the type of an analysis item.

For example, when the particle measuring device 10 is used for an HIV test, CD4 antigen and CD8 antigen on the surface of cells are fluorescently labeled. Then, the measurement unit 75 of the particle measuring device 10 receives a fluorescence generated from the fluorescence-labeled antibody bound to CD4 antigen, and a fluorescence generated from the fluorescence-labeled antibody bound to CD8 antigen.

On the basis of the intensity of the detection signals, the controller 71 of the particle measuring device 10 obtains, as the number of CD4-positive T-lymphocytes, the number of cells that have CD4 antigen expressed on the cell surfaces, and obtains, as the number of CD8-positive T-lymphocytes, the numbers of cells that have D8 antigen expressed on the cell surfaces. Then, the controller 71 calculates a positive rate obtained by dividing the number of CD4-positive T-lymphocytes by the number of CD8-positive T-lymphocytes. On the basis of the calculated cell number and positive rate, the controller 71 determines whether or not the subject is contracted with HIV.

For example, when the particle measuring device 10 is used for a transplantation test of hematopoietic stem cells, CD34 antigen on the surfaces of cells are fluorescently labeled. Then, the measurement unit 75 receives a fluorescence generated from the fluorescence-labeled antibody bound to CD34 antigen. On the basis of the intensity of the detection signal, the controller 71 obtains the number of cells that have CD34 antigen expressed on the cell surfaces, as the number of CD34-positive cells, i.e., the number of hematopoietic stem cells. Then, the controller 71 calculates the ratio of the hematopoietic stem cells. In hematopoietic stem cell transplantation, hematopoietic stem cells provided by a donor is transplanted to a patient. The performance of such a therapy is significantly dependent on the number of hematopoietic stem cells transplanted to the patient. Therefore, a doctor or the like can utilize the number or ratio of hematopoietic stem cells obtained by the particle measuring device 10, in determination of whether or not the donor is appropriate or whether or not the therapy is successful.

For example, when the particle measuring device 10 is used in tests for leukemia and lymphoma, various markers are fluorescently labeled in accordance with the hemocytes as the test target. When hemocytes of the T cell system, the B cell system, and the myeloid system are test targets, markers shown in Table 1 below are fluorescently labeled. When other hemocytes are test targets, other markers shown in Table 1 below are fluorescently labeled.

[Table 1]

| T cell system | B cell system | Myeloid system | Other |
|---|---|---|---|
| CD1 | CD19 | CD13 | CD10 |
| CD2 | CD20 | CD14 | CD38 |
| CD3 | CD22 | CD33 | CD56 |
| CD4 | CD23 | CD34 | CD58 |
| CD5 | CD79a | CD64 | HLA-DR |
| CD7 | IgM (μ chain) | CD65 | CD41/CD61 |
| CD8 | κ/λ (L chain) | CD117 | CD42b |
| TCR | FMC7 | MPO | CD235a |

Also in this case, the measurement unit 75 receives a fluorescence generated from the fluorescence-labeled antibody bound to the antigen present on the surface or in the cytoplasm of the cells. On the basis of the intensities of detection signals based on a plurality of fluorescences, the controller 71 obtains the number of cells of each system, i.e., the numbers of lymphocytes, monocytes, T-cells, and B-cells. In addition, the controller 71 obtains the numbers of cells at maturation stages, i.e., the number of juvenile cells and the number of mature cells. In this manner, on the basis of combinations of a plurality of antigens expressed in a single cell, the controller 71 obtains the number of cells of each system and the number of cells at each maturation stage. Therefore, a doctor or the like can utilize the cell number obtained by the particle measuring device 10, in determination of which type of cell has become tumorigenic.

INDUSTRIAL APPLICABILITY

The present invention can be suitably used, for example, as a particle measuring device and a particle measuring method for measuring particles.

DESCRIPTION OF THE REFERENCE CHARACTERS

| | |
|---|---|
| 10 | particle measuring device |
| 11 | sample |
| 20 | flow cell |
| 30 | light applicator |
| 41 | objective lens |
| 42 | condenser lens |
| 43 | light guiding lens |
| 43a | light-entry-side end surface |
| 50 | light transmission part |
| 50a | light-entry-side end surface |
| 51 | optical fiber |
| 61 | light detector |
| 201, 202, 203 | light guiding lens |
| 201a, 202a, 203a | light-entry-side end surface |
| 210, 220, 230 | light transmission part |
| 210a, 220a, 230a | light-entry-side end surface |
| 211, 221, 231 | optical fiber |
| 300, 400, 500 | light detector |
| 300a, 400a, 500a | base plate |
| 301, 401, 501 | collimator lens |
| 321 to 326, 421 to 423, 521 to 526 | second condenser lens |
| 331 to 336, 431 to 433, 531 to 536 | light receiving element |

**Claims**

1.  A particle measuring device comprising:

    a flow cell in which a sample including a particle flows;
    an irradiator configured to irradiate an irradiation light to the sample flowing in the flow cell;
    a condenser lens to condense a light generated from the particle included in the sample which is irradiated by the irradiation light;
    a light transmitter which is formed by a plurality of optical fibers being bundled, and which the light having passed through the condenser lens enters; and
    a light detector configured to receive the light transmitted by the light transmitter and output a detection signal.

2.  The particle measuring device according to claim 1, wherein
    the condenser lens condenses the light generated from the particle, within a light-entry-side end surface of the light transmitter.

3.  The particle measuring device according to claim 1, wherein
    the condenser lens condenses the light generated from the particle to a light-entry-side end surface of the light transmitter, such that the light has an area having a diameter greater than that of a light entry end of each of the optical fibers and extending across a plurality of light entry ends of a plurality of the optical fibers.

4.  The particle measuring device according to claim 1, further comprising
    a light guiding lens configured to guide the light condensed by the condenser lens, to a light-entry-side end surface of the light transmitter.

5.  The particle measuring device according to claim 4, wherein
    the light guiding lens enlarges a diameter of the light condensed by the condenser lens, and guides the light to the light-entry-side end surface of the light transmitter.

6.  The particle measuring device according to claim 5, wherein

the light guiding lens causes the light condensed by the condenser lens to enter, as a collimated light, the light-entry-side end surface of the light transmitter.

7. The particle measuring device according to claim 4, further comprising
an objective lens configured to allow the light generated from the particle to pass, wherein
a distance between a rear-side principal plane of the objective lens and a principal plane of the condenser lens, and
a distance between the principal plane of the condenser lens and a light-entry-side end surface of the light guiding lens are each set to a focal length of the condenser lens.

8. The particle measuring device according to claim 4, wherein
the condenser lens converges the light generated from the particle, to a light-entry-side end surface of the light guiding lens, and
the light guiding lens causes the light converged by the condenser lens to enter the light-entry-side end surface of the light transmitter, such that the light has an area having a diameter greater than that of a light entry end of each of the optical fibers and extending across a plurality of light entry ends of a plurality of the optical fibers.

9. The particle measuring device according to claim 4, wherein
a condition for a focal length of the light guiding lens is represented by a formula below,

[Math 1]

$$f2 < \frac{\sqrt{m} \times b}{2 \times NA1}$$

where f2 is the focal length of the light guiding lens, m is the number of the optical fibers of the light transmitter, b is an outer diameter of each of the optical fibers in the light transmitter, and NA1 is a numerical aperture of the condenser lens.

10. The particle measuring device according to claim 4, wherein
the light guiding lens is a graded index lens in which a refractive index is reduced in accordance with increase in a distance from a central axis thereof.

11. The particle measuring device according to claim 4, wherein
the light guiding lens is adhered to light entry ends of a plurality of the optical fibers.

12. The particle measuring device according to claim 1, wherein
the light detector includes:

a light receiving element configured to receive the light transmitted by the light transmitter; and
an optical system disposed between the light transmitter and the light receiving element and configured to guide the light transmitted by the light transmitter, to the light receiving element.

13. The particle measuring device according to claim 12, wherein
the optical system includes a collimator lens configured to convert lights outputted from a plurality of the optical fibers of the light transmitter, into a collimated light.

14. The particle measuring device according to claim 13, wherein
the optical system includes a second condenser lens disposed between the collimator lens and the light receiving element, and
the second condenser lens condenses the light converted into the collimated light by the collimator lens, and guides the light to the light receiving element.

15. The particle measuring device according to claim 14, wherein
the collimator lens is configured to be able to take in lights outputted from all of the optical fibers forming the light transmitter, and
the second condenser lens guides the lights taken in by the collimator lens, to the light receiving element.

16. The particle measuring device according to claim 1, wherein
the irradiator irradiates, to the sample, a plurality of the irradiation lights having wavelengths different from each other, and
the particle measuring device comprises:

> a plurality of the light transmitters respectively corresponding to the plurality of the irradiation lights; and
> a plurality of the light detectors configured to receive the lights respectively transmitted by the plurality of the transmitters and output detection signals.

17. The particle measuring device according to claim 16, wherein
the plurality of the light detectors are set on base plates different from each other.

18. The particle measuring device according to claim 16, wherein
the irradiator irradiates the plurality of the irradiation lights to respective positions different from each other in a flow direction of the sample.

19. The particle measuring device according to claim 16, wherein
the condenser lens is configured to inhibit chromatic aberration with respect to the lights respectively generated due to the plurality of the irradiation lights.

20. The particle measuring device according to claim 1, wherein
the sample includes a cell as the particle, and
a plurality of markers different from each other of the cell are respectively stained by a plurality of fluorescent dyes that generate, due to the irradiation light, fluorescences having wavelengths different from each other.

21. A particle measuring method comprising:

> causing a sample including a particle to flow in a flow cell;
> irradiating an irradiation light to the sample flowing in the flow cell;
> condensing a light generated, by irradiation of the irradiation light, from the particle included in the sample;
> causing the condensed light to enter a light entry end of an optical fiber bundle formed by a plurality of optical fibers being bundled; and
> receiving the light transmitted by the optical fiber bundle and outputting a detection signal.

22. The particle measuring method according to claim 21, wherein
the causing of the light to enter the light entry end of the optical fiber bundle includes enlarging a diameter of the condensed light and causing the resultant light to enter the light entry end of the optical fiber bundle.

FIG.1

EMBODIMENT 1

10

Z
Y ⊗ → X

FIG.2

EMBODIMENT 2

10

30

12

41

42

50

43a    50a    50b    61

20

21

11

43

51

52

Z

Y    X

FIG.3

62 — PRETREATMENT DEVICE ◄- - - - - - - - -

13

11

10

PARTICLE MEASURING DEVICE

71 — CONTROLLER

72 — STORAGE UNIT ◄——► MEASUREMENT UNIT — 75

73 — DISPLAY UNIT ◄——► INPUT UNIT — 74

FIG.4

FORWARD SCATTERED LIGHT

SIDE SCATTERED LIGHT AND FLUORESCENCE

FIG.5

FIG.6A

LIGHT FROM CONDENSER LENS 42

201a 201b 210a

201 210

A M

Z
Y⊗→X

FIG.6B

ΔD

LIGHT FROM CONDENSER LENS 42

θ1 201b 210a

201a

201 210

A M

Z
Y⊗→X

FIG.7A

NUMBER OF
OPTICAL
FIBERS m

FIG.7B

FIG.7C

FIG.8

FIG.9

FIG.10

FIG.11

FIG.12

FIG.13

CELL

FLUORESCENCE-LABELED
ANTIBODY F11
TO MARKER P11 ◄-------- ►―O ◄―·―·―·―·― LIGHT HAVING WAVELENGTH $\lambda 1$
―·―·―·―·―·► FLUORESCENCE HAVING WAVELENGTH $\lambda 11$

FLUORESCENCE-LABELED
ANTIBODY F15
TO MARKER P15 ◄-------- ►―O ◄―·―·―·―·― LIGHT HAVING WAVELENGTH $\lambda 1$
―·―·―·―·―·► FLUORESCENCE HAVING WAVELENGTH $\lambda 15$

FLUORESCENCE-LABELED
ANTIBODY F21
TO MARKER P21 ◄-------- ►―O ◄―·―·―·― LIGHT HAVING WAVELENGTH $\lambda 2$
―·―·―·―·► FLUORESCENCE HAVING WAVELENGTH $\lambda 21$

FLUORESCENCE-LABELED
ANTIBODY F23
TO MARKER P23 ◄-------- ►―O ◄―·―·―·― LIGHT HAVING WAVELENGTH $\lambda 2$
―·―·―·―·► FLUORESCENCE HAVING WAVELENGTH $\lambda 23$

FLUORESCENCE-LABELED
ANTIBODY F31
TO MARKER P31 ◄-------- ►―O ◄―·―·―·― LIGHT HAVING WAVELENGTH $\lambda 3$
―·―·―·―·► FLUORESCENCE HAVING WAVELENGTH $\lambda 31$

FLUORESCENCE-LABELED
ANTIBODY F36
TO MARKER P36 ◄-------- ►―O ◄―·―·―·― LIGHT HAVING WAVELENGTH $\lambda 3$
―·―·―·―·► FLUORESCENCE HAVING WAVELENGTH $\lambda 36$

FIG.14

## INTERNATIONAL SEARCH REPORT

| International application No. |
|---|
| PCT/JP2019/002505 |

A. CLASSIFICATION OF SUBJECT MATTER
Int. Cl. G01N15/14(2006.01)i, G01N33/543(2006.01)i

According to International Patent Classification (IPC) or to both national classification and IPC

B. FIELDS SEARCHED

Minimum documentation searched (classification system followed by classification symbols)
Int. Cl. G01N15/14, G01N33/543

Documentation searched other than minimum documentation to the extent that such documents are included in the fields searched
Published examined utility model applications of Japan    1922-1996
Published unexamined utility model applications of Japan  1971-2019
Registered utility model specifications of Japan          1996-2019
Published registered utility model applications of Japan  1994-2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br><br>A | JP 62-44650 A (CANON INC.) 26 February 1987, page 1, left column, line 3 to page 3, lower left column, line 5, fig. 1 (Family: none) | 1-3, 21<br>4-7, 10, 12-20, 22<br>8-9, 11 |
| Y | JP 5-48413 B2 (CANON INC.) 21 July 1993, page 1, left column, line 2 to page 2, right column, line 35 (Family: none) | 4-7, 10, 14-15, 22 |
| Y | JP 9-79969 A (TOA MEDICAL ELECTRONICS CO., LTD.) 28 March 1997, paragraphs [0002]-[0042], fig. 1-4 & US 5822062 A, column 1, line 6 to column 8, line 12, fig. 1-4 | 4-7, 10, 14-15, 22 |

☒ Further documents are listed in the continuation of Box C.          ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>10.04.2019 | Date of mailing of the international search report<br>23.04.2019 |
|---|---|
| Name and mailing address of the ISA/<br>    Japan Patent Office<br>    3-4-3, Kasumigaseki, Chiyoda-ku,<br>    Tokyo 100-8915, Japan | Authorized officer<br><br><br>Telephone No. |

Form PCT/ISA/210 (second sheet) (January 2015)

**INTERNATIONAL SEARCH REPORT**

| International application No. |
| --- |
| PCT/JP2019/002505 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 61-182549 A (OMRON TATEISI ELECTRONICS CO.) 15 August 1986, page 1, left column, line 3 to page 4, left column, line 10, fig. 1 (Family: none) | 7, 14-15 |
| Y | JP 2011-95181 A (SYSMEX CORP.) 12 May 2011, paragraphs [0026]-[0040], fig. 3-5 & US 2011/0102792 A1, paragraphs [0031]-[0049], fig. 3-5 & EP 2317302 A2 & CN 102053056 A | 7, 14-15 |
| Y | JP 2013-160672 A (SONY CORP.) 19 August 2013, paragraphs [0018]-[0042], fig. 6 & US 2013/0200272 A1, paragraphs [0039]-[0071], fig. 6 & CN 103245643 A | 12-13, 14-20 |
| Y | JP 10-253624 A (TOA MEDICAL ELECTRONICS CO., LTD.) 25 September 1998, paragraphs [0021]-[0038], fig. 1 (Family: none) | 14-15 |
| Y | JP 2011-169916 A (SYSMEX CORP.) 01 September 2011, paragraph [0028], fig. 1 (Family: none) | 14-15 |
| A | JP 2006-292769 A (BAY BIOSCIENCE KK) 26 October 2006, entire text, all drawings (Family: none) | 1-22 |
| A | JP 61-51569 A (CANON INC.) 14 March 1986, entire text, all drawings (Family: none) | 1-22 |
| A | JP 2005-536740 A (COULTER INTERNATIONAL CORPORATION) 02 December 2005, entire text, all drawings & US 2004/0036874 A1, entire text, all drawings & EP 1540310 A1 | 1-22 |
| A | JP 2014-228409 A (SHARP CORP.) 08 December 2014, entire text, all drawings (Family: none) | 1-22 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6683314 B